(19) Europäisches Patentamt

European Patent Office

Office européen des brevets

(11) **EP 1 934 994 B1**

(12) **EUROPEAN PATENT SPECIFICATION**

(45) Date of publication and mention
of the grant of the patent:
**18.01.2012 Bulletin 2012/03**

(51) Int Cl.:
***H01G 4/35*** *(2006.01)* ***A61N 1/375*** *(2006.01)*

(21) Application number: **06814689.3**

(22) Date of filing: **15.09.2006**

(86) International application number:
**PCT/US2006/035928**

(87) International publication number:
**WO 2007/035445 (29.03.2007 Gazette 2007/13)**

(54) **IMPLANTABLE CO-FIRED ELECTRICAL FEEDTHROUGHS**

IMPLANTIERBARE ZUSAMMEN AUSGELÖSTE ELEKTRISCHE DURCHSPEISUNGEN

TROUS D'INTERCONNEXION ELECTRIQUES, COFRITTES, IMPLANTABLES

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **15.09.2005 US 227342**

(43) Date of publication of application:
**25.06.2008 Bulletin 2008/26**

(73) Proprietor: **Medtronic, Inc.
Minneapolis, MN 55432-5604 (US)**

(72) Inventors:
• **BURDON, Jeremy W.
Phoenix, AZ 85054 (US)**
• **YAMAMOTO, Joyce K.
Maple Grove, Minnesota 55311 (US)**

• **NYGREN, Lea A.
Bloomington, Minnesota 55438U (US)**
• **WOLF, William D.
St. Louis Park, Minnesota 55426 (US)**

(74) Representative: **Hughes, Andrea Michelle
Dehns
St Bride's House
10 Salisbury Square
London
EC4Y 8JD (GB)**

(56) References cited:
**EP-A2- 1 178 713        US-A- 5 277 724
US-A- 5 855 995          US-A1- 2002 050 316
US-A1- 2003 218 870      US-B1- 6 306 511
US-B1- 6 488 795**

## Description

## FIELD

This disclosure relates to co-fired electrical interconnects for implantable medical systems and devices, wherein said systems and devices are devoid of high-temperature brazed couplings

## BACKGROUND

Implantable medical devices (IMDs) are steadily being miniaturized and their functionality is increasing. This is driving size and cost reduction of all IMD components including the electrical feedthrough, where it is desirable to reduce device size while increasing the number of electrical feedthroughs and interconnects. Feedthroughs are required that operate in potted and unpotted conditions (requiring biocompatibility), and in addition the functionality of the implantable device often requires the device operate at a voltage-bias, which puts body fluid-contacting electrical connections under electrochemical stress which can possibly result in erroneous operation and/or device failure. In addition, there is a growing need to reduce the cost of the components used in medical devices. Current electrical feedthroughs are costly due to the many piece-parts and multiple processes required to fabricate and assemble these parts into a functional component.

For additional background on the general field of endeavor and context of the present invention, see U.S. Pat. No. 6,743,534 issued to Lautzenhiser et al. on 1 June 2004 and entitled, "Self-constrained low temperature glass-ceramic unfired tape for microelectronics and methods for making and using the same ",

An excerpt from the Background portion of the '534 patent follows to aid the reader in understanding certain aspects of the present invention as well as related and inherent aspects thereof.

The co-sintering or firing of laminated ceramic tapes is a well-known module manufacturing method in the microelectronics industry. The phrase low-temperature co-fired ceramic (LTCC) refers to a technology for forming multilayered ceramic circuits. In this approach, a tape is formed from glass and ceramic powders uniformly dispersed in an organic binder. Typically, two or more layers of this tape are laminated together to form a circuit. To form electrical connections from one layer of tape to the next, via holes are punched through the tape and filled with a thick-film conductor paste, for example as described in U.S. Pat. No. 4,654,095 to Steinberg. In addition, other exemplary prior art includes U.S. Pat. No. 4,641,425 to Dubuisson et al. entitled, "Method of Making Alumina Interconnection Substrate for an Electronic Component" and U.S. Pat. No. 4,910,643 to Williams entitled, "Thick Film Multi-layer, Ceramic Interconnected Circuit Board." Further prior art also includes U.S. Pat. No. 4,464,420 to Taguchi et al. entitled, "Ceramic Multilayer Circuit Board and a Process for Manufacturing Same," U.S. Pat. No. 5,356,841 to Mitzutani et al. entitled, "Glass-Ceramic Composite" U.S. Pat. No. 5,831,810 to Bird et al. entitled "Electronic Component Package with Decoupling Capacitors Completely within Die Receiving Cavity of a Substrate," and US 5 855 995.

Now continuing with reference to the '095 patent to Steinberg, in a next step, thick-film pastes (dispersions of metallic, ceramic or glass powders in volatilizable organic vehicles) that will form components of electronic circuits, such as conductors or resistors, are then screen-printed onto the tape. After all of the layers of tape necessary to form the completed circuit have been prepared, the pieces of tape are aligned to ensure that via connections from one layer will make contact with conductor traces or via holes on the next. The layers of tape are then laminated with a combination of heat and pressure to form a single green body, i.e., a structure that is held together by organic binders, such as polyvinyl butyral or acrylate materials. In order to form the final ceramic body, the green body is fired in a firing profile that typically reaches a peak temperature of about 850 degrees Celsius to about 900 degrees Celsius before returning to ambient temperature. In a range of temperatures between about 350 degrees Celsius and about 450 degrees Celsius, the organic binders that give the green body strength are volatilized or burned out. To give the volatilized gases sufficient time to escape, the ramp rate (change in temperature per unit time) of the profile is often reduced in this temperature range.

Above the burnout temperature, the ramp rate of the firing profile is increased and the part is heated until reaching the peak firing temperature. The LTCC tape typically contains a significant amount of a glass with which a glass softening point is associated. The glass and ceramic powders will begin to sinter into a dense body when the temperature is above the softening point of the LTCC glass, so the peak firing temperature of the tape is typically 100 degrees Celsius to 200 degrees Celsius above the glass softening point. The thick-film conductor and resistor materials used in the circuit body will undergo a similar metamorphosis from organically bound powders into dense sintered structures. After allowing the parts to remain at the peak firing time to reach an adequately dense body, the parts are cooled to room temperature.

Manufacturing of LTCC tapes is typically performed using tape casting techniques, such as those described in U.S. Pat. No. 5,821,181 of Ursula, et al. In this method, ceramic slurry (a mixture of the inorganic and organic components of the tape before drying) is deposited on top of a polyester film or carrier using a doctor blade. One disadvantage of using tape casting techniques for tape manufacturing is the difficulty of thickness control as the tape becomes thinner and thinner. More specifically, thickness, accuracy and variance become uncertain when casting under two mils (50 microns), a measurement which refers to the gap between the blade and the backing as the wet slurry passes through. Therefore, control of the layer thickness, especially of inner layers, becomes difficult and often inaccurate.

While accurate casting of individual layers is achievable, the method described in U.S. Pat. No. 5,102,720 for drying the tapes individually and subsequently laminating them together is uneconomical. Thus, methods which involve drying individual layers and lamination with heat and pressure, or casting a subsequent layer on top of a dry layer, not only introduce significant costs to the manufacturing process, but also limit product yields.

Other manufacturing methods include dipping a moving carrier film in a slurry to create a meniscus between the carrier film and the slurry. However, the meniscus created by capillary forces between the wet organic binder and the film causes it to stick to the surface of the polyester film. As in other methods, drying one layer at a time and then casting a wet layer on top of a dry layer or subsequent heat lamination are needed. Because of the disadvantages with known methods for manufacturing LTCC tapes, there remains a need in the art for an improved, economical method for fabricating LTCC tapes which will maximize product yield and permit tight control of layer thickness.

The LTCC technology has advanced beyond the microelectronic circuit industry and is currently in use for a variety of applications. One important attribute of LTCC is the ability to create three-dimensional structures using multiple layers of tape. The biomedical device industry, for example, uses LTCC for the manufacturing of cavities and channels for moving part pumps used in in-situ drug delivery systems. Biological test modules have been realized which facilitate the automatic testing of biological and chemical materials.

In the telecommunications industry, there is a need for integrated opto-electronic modules. LTCC offers the simplicity of being able to co-sinter optical fibers together with the driving electronics. The co-firing of meso-scale structures containing metallization, cavities, vias, and channels is thus an appealing feature of LTCC.

LTCC meso-systems are small packages capable of handling at least two media, such as electricity and fluids, by means of sensors, actuators, interconnection, control and/or signal processing. Miniaturization is one of the biggest drivers of this technology, thus allowing systems in package (SIP), in which several components are inserted into a monolith.

An attractive feature of LTCC tapes is the possibility for making cavities for the placement of integrated circuits within. For example, an electronic module can be fabricated that contains a cavity, a metallic via, and a metallic line trace on the surface of a ceramic monolith.

Cavities allow a module to retain a low profile, and according to certain prior approaches a lid can be placed on top for hermeticity. However, during surface or sacrificial constrained sintering, as explained below, the cavity walls exhibit a phenomenon called necking, a vertical curvature from the top surface interface to the bottom of the fired substrate surface. During sintering of sacrificially constrained structures, there is a stress distribution due to the shear and in-plane tensile stresses from top to bottom. It has been shown that stresses are significantly higher at the constrained interface. Moving along the z-axis towards the middle of the fired substrate, there are fewer constraining forces that counteract the in-plane tensile stresses. Therefore, there is significantly more densification in the middle of the monolith, which causes the vertical curvature. Furthermore, as a consequence of the higher stress distribution at the interface, delamination or buckling is usually present. The aforementioned properties are undesirable, especially when constructing cavities or other precision features in the ceramic structures.

Despite the numerous applications of LTCC technology, the LTCC process has several disadvantages. First, there are significant changes in the dimensions of the ceramic monolithic structure during sintering. More specifically, when the constituent powders of the LTCC structure densify during traditional unconstrained or free sintering, shrinkage occurs in all dimensions. Typically, the shrinkage of the tape across its width or length (the x- or y-directions) will be nearly identical and only slightly different from the shrinkage through the thickness of stack-up of tape layers (the z-direction). Usually, the dimensions of the structure after firing will be about 84% to 87% of the size in the unfired green state. This change and the associated variations result in several disadvantages to the use of conventional LTCC technology that present challenges for the use of LTCC technology for certain applications (e.g., applications requiring a long-term hermetic seal, especially in the presence of corrosive fluids or the like).

During firing, the shrinkage uncertainty of the LTCC causes the external features to vary with respect to the intended nominal position. Artworks used for post-firing processes, such as the printing of post-fired conductors or resistors, or for printing solder on conductors, are based on the intended nominal position. Excessive distance between the actual fired position of a circuit feature and the nominal position can cause circuit failures if, for example, there is failure to make adequate electrical contact, which may result from lack of via connections or misalignments between layers due to non-uniform shrinkage. Alternatively, although artwork features may be enlarged to allow for such shrinkage variation, decreased circuit density may result.

Previously pressure-assisted sintering and the application of external loads to ceramic tape modules are described in U.S. Pat. No. 4,340,436. The use of mechanical clamping on the periphery of a ceramic panel to contain its x-y dimensions is also discussed in the prior art (*see* European Patent No. 0 243 858).

These types of approaches present several potential problems and disadvantages to the manufacturer. Because the presence of the platen may cause functional defects in any conductors or resistors which are in direct contact with the surface of the LTCC, the platen contact geometry must be carefully controlled and aligned with the green tape. Use of mechanical clamping techniques may require different platen designs for different circuits or geometry for an article fabricated using LTCC technology. Finally, a separate platen must be used for each constrained structure being fired in a batch.

Alternatively, the use of porous contact sheets attached to the LTCC panels that are easily removed after sintering is described in U.S. Pat. No. 6,139,666. Additionally, as described in U.S. Pat. No. 6,205,032 and U.S. Pat. No. 6,560,860 entitled, "Low Temperature Co-Fired Ceramic with Improved Registration," describes the use of a constraining ceramic core that constrains the attached layers using subsequent firings has been attempted.

A further technique for constraining the x-y geometry of LTCC circuits involves laminating sacrificial constraining tape layers onto the top and bottom surfaces of the LTCC circuit body. This technique has been described, for example, in U.S. Pat. Nos. 5,085,720; 5,254,191; 5,383,474; and 5,474,741, all by Mikeska, et al. The sacrificial tape layers are formed from porous, high temperature refractory ceramic powder that by itself will not sinter during the LTCC firing process. Since the sacrificial tape does not sinter and densify during the firing profile, it maintains the geometry of its green state.

However, in order for the sacrificial refractory tape to constrain the x-y geometry of underlying LTCC tape, at least two conditions should be met. First, there must be sufficient friction between the two tape materials to mechanically link the materials. Second, glassy components of the LTCC tape that could dissolve the refractory component of the sacrificial tape during the LTCC firing profile, thus allowing it to sinter and densify, must not saturate the sacrificial tape layer.

All of the aforementioned external constraint approaches have significant drawbacks. For example, pressure-assisted sintering and peripheral constraining require special adaptation of the furnace or the need for external equipment to mechanically prevent shrinkage of the ceramics. Other methods require the creation of refractory ceramic porous molds to form the tape for cavities.

Finally, several potential problems exist for manufacturers using sacrificial tape processes. After firing, the sacrificial tape layer must be removed from the circuit body sufficiently completely to not interfere with subsequent manufacturing processes, but not so aggressively as to damage the remaining LTCC body. Like the platen of the mechanical clamping technique, the sacrificial tape may be incompatible with conductors or resistors that may be placed on the surface of the LTCC circuit body. Therefore, these surface features must be printed and fired after removal of the sacrificial layer, which increases the number of processing steps on the manufacturing line and also results in increased cost of successive firings (furnace costs). From the standpoint of process yield and process simplicity, it would have been preferable to print these features on green tape and co-fire them with the rest of the circuit body. Further, because the sacrificial tape has virtually no mechanical strength after firing, it cannot be incorporated into the body of the LTCC circuit. This limits the thickness of bodies that can be constrained with this method, as the degree of constraint deteriorates with an increase in the distance from the constraining layer. Finally, contact sheets of refractory ceramic sacrificial tape have the potential for surface contamination of the LTCC tape, and the removal or dusting and waste of the sacrificial layer contribute to and reflect on the individual module cost.

SUMMARY

The present invention provides a miniaturised hermetic electrical interconnect as defined by claim 1.

Unlike some prior art methods and apparatus, certain embodiments of the present invention involve use of low temperature co-fired ceramic (LTCC), high temperature co-fired ceramic (HTCC) and combinations of both LTCC and HTCC fabrication and processing methods and structures. In general, such ceramic structures are formed using particles of sinterable, inorganic oxides such as ceramics and glass-ceramics particles, and processed in layer form to allow integration of electrical conductors in the x, y and Z planes to form a substantially monolithic 3-dimensional integration circuit. In general, the inorganic oxides comprise a high-temperature dielectric such as alumina ($Al_2O_3$), Silica ($SiO_2$) or Zirconia ($ZrO_2$) or mixtures thereof, and glass suspended in an organic (polymer) binder. This material is derived from a precursor ceramic slurry, comprised of the various inorganic components dispersed in a mixture of polymer and solvent. This material is formed into thin-sheets using a 'tape casting process' utilizing a blade, a well-known and established process

Individual sheets (or segments of tape) are printed with a metallized paste and other circuit patterns, stacked on each other, laminated together and subjected to a predetermined temperature and pressure regimen, and then fired at an elevated temperature(s) during which the majority of binder material(s) (present in the ceramic) and solvent(s) (present in the metallized paste) vaporizes and/or is incinerated while the remaining material fuses or sinters. Typically materials suitable for use as co-fireable conductors are Platinum, Iridium, Platinum-Iridium alloys, Silver, Gold, Palladium, Silver-Palladium or mixtures thereof, or Tungsten, Molybdenum and/or Moly-manganese or other suitable materials are typically constitute the metallized paste. Thus, the green sheets are patterned and then stacked and aligned in an appropriate laminated configuration. The stacked laminates are then fired at temperatures of about 600 to about. 800 degrees Celsius (for LTCC) and about 1300 to about 1600 degrees Celsius (for HTCC). In most cases, the debinderization step is performed in an oxidizing atmosphere (air) to assure decomposition of the organic components. The subsequent sintering phases of the firing process may proceed in an oxidizing or inert atmosphere depending on the conductor system. For example, an LTCC that utilizes Gold or Gold-Palladium conductors or an Alumina HTCC system that uses Platinum will be fired in air, whereas a Tungsten-Molybdenum system will likely require an inert atmosphere such as N2/H2 mixture. In general, an LTCC system will employ a lower melting-point conductor metallization such as Gold or Silver, where HTCC technology typically employs high-melting point refractory metal pastes as conductors.

According to certain aspects of the present invention a family of low-cost, miniaturized, hermetic electrical feedthrough assemblies suitable for implantation within tissue and/or in direct or indirect contact with diverse body fluids is provided. Such miniaturized, hermetic electrical feedthrough assemblies are made by forming an electrical interconnects in one or more ceramic green-sheet layer(s), stacking and laminating the layers together, and sintering them together to form a substantially monolithic dielectric structure having at least one embedded metallization pathway extending through the structure. Said metallization pathway provides communication of electrical signals in a variety of medical applications, including those requiring voltage-bias. The assemblies hermetically seal to a ferrule or the periphery of an aperture formed in a portion of a housing of an IMD, for example, from internal circuitry to external circuitry and/or components and can be directly and/or indirectly exposed to living tissue and body fluids. Alternatively, the ferrule can be sealingly disposed in an aperture formed in an enclosure for an electrochemical cell, physiologic sensor and the like. A diffusion bond hermetically couples the dielectric structure to the interior of the ferrule and/or an adjacent lower support member. In some embodiments, in addition to the diffusion bond a high temperature braze material provides an additional fluid seal between the ferrule and the dielectric structure.

The feedthrough components according to the invention are uniquely adapted for hermetic insertion into enclosures for electrochemical cells configured for implantation in so-called active IMDs (e.g. primary and secondary batteries, capacitors, etc.), diverse implantable physiologic sensors or capsules for such sensors (e.g. pressure, temperature, electrogram, flow, pH, blood chemistry, impedance, saturated oxygen and surrogates therefor, etc.). To improve ease of conductive coupling to one or both of the opposing sides of a feedthrough constructed according to the invention, one or more bonding, or capture, pads can be affixed thereto. Such a capture pad can comprise a plate that is post-fired to bond to a surface via of a feedthrough or a volume of deposited electrically conductive powder of noble metals (e.g., platinum, iridium, Palladium, gold and alloys thereof) or refractory metals (e.g., niobium, tungsten, molybdenum, and alloys thereof) that are co-fired or post-fired during fabrication, deposited as a thin-film using most-any suitable thin-film deposition or plating technology. Optionally, an elongated conductor (e.g. a pin or ribbon, wire, or connector-block) can be coupled to a capture pad for connection to remote circuitry or components.

In addition, certain embodiments of the invention implement more than a single conductive path between opposing sides of a feedthrough assembly. For example, in the event that the conductive traces couple to a rapid high energy discharge circuit such as an implantable defibrillator a plurality of individual conductive pathways can be utilized to conduct the defibrillation waveform. Such a waveform can comprise a biphasic waveform having an amplitude on the order of several hundred volts. One advantage of this aspect of the invention relates to the fault tolerance two or more conductive paths provide. In addition to or in lieu of the foregoing, the size and/or shape of the metallized vias can be adjusted for an intended application. According to this aspect of the invention, one or more conductors intended to carry large amounts of electrical current can be designed with a larger diameter.

By the same token, in a multi-polar feedthrough structure for an implantable cardioverter-defibrillator (ICD), the very low power circuitry used for cardiac pacing can couple to relatively thin or smaller metallized vias while the very high power defibrillation therapy delivery circuits can couple to relatively large metallized vias. Also, one or more conductors coupled to very low power remote physiologic sensors or a telemetry antenna can couple to yet another size metallized via. A multi-polar feedthrough array can include a linear or non-linear array of capture plates or metallized vias. Of course, a multi-polar feedthrough can also implement a regular distribution, an irregular distribution, or a combination of regular and irregular distribution over the exposed surface of a co-fired ceramic feedthrough assembly.

The following table shows the bulk resistivity (☐) of pure metals that may be employed for the invention

| Metal | Bulk Resistivity (uOhm.cm) |
|---|---|
| Niobium | 16 |
| Platinum | 10 |
| Tungsten | 5.4 |
| Gold | 2.2 |
| Copper | 1.69 |
| Silver | 1.63 |

Referring now to equation X.

## Equation X :

$$Reff = \square \ L/A$$

Where Reff is the effective resistance of the structure, $\square$ is the bulk resistivity of the pure metal, L is the physical length of the conductor and A is the cross-sectional area of the conductor, it can be realized that although in general, the value for $\square$ for the co-fired metallization is 10-100x lower than the pure metal, the reduction in length and / or the use of multiple conductor pathway allows Reff to be reduced. For example, where as in a conventional FT the pin conductor may be 50-100mil, the co-fire electrical FT may be as small as 20-30mil. In addition, multiple co-fire FT vias may be electrically connected in parallel to drastically reduce the effective resistance, while still maintaining the desired lower profile.

This concept of using multiple vias in illustrated in Figure 13, which depicts the external to internal to external co-fire FT architecture, utilizing three dielectric layers (with alternating three-via arrangement), two nominally round-shaped interconnect pads to interconnect the three-via electrical interconnects internally, and two external interconnect pads (top and bottom of the device).

In terms of the shape of a metallized via and/or a capture plate affixed to a via in order to enhance accuracy of automated and manual assembly a surface portion of a via or capture plate can be designed with a recognizable characteristic shape, size, color and/or the like so that, particularly for multi-polar feedthrough assemblies, the ultimate electrical couplings are accurately and reliably secured with the aid or human- or machine-vision assisted pick and place component assembly equipment.

With respect to hermeticity, testing of a plurality of three layer co-fired feedthrough units revealed that approximately 30 pounds of force was required produce dislodgement of feedthroughs that were brazed using an annular gold perform or diffusion-bonded using thin-film Niobium interlayer, into a titanium ferrule fitted into an aperture formed in a titanium plate. In fact this force exceeded the tensile strength of the co-fire ceramic insulator, as the device always fractured within the ceramic, not at the ceramic-ferrule interface. In addition, during leak testing such units were immersed in a saline solution maintained at approximately 37 degrees Celsius (approximate body temperature) and approximately 95 degrees Celsius for thirty days both with an applied nominal bias voltage (2.2 V, 4.0 V) and without any bias voltage and no leakage was detected. The foregoing testing was performed on unipolar feedthrough units comprising three ceramic layers and two interlayers of a platinum paste co-fired at approximately 1500 degrees for about four hours.

Structures according to the invention can be aligned using fiducial marks, laser or other optical mechanisms or the like. Typically the lateral side wall portions of a feedthrough according to the invention are aligned substantially parallel to adjacent and opposing side wall portions; however, the side wall portions can be fabricated at any reasonable angle relative to adjoining structure (s) and/or can be fabricated with a varying topography. In addition to or in lieu of such fabrication, one or more surfaces of a co-fired structure can be mechanically altered prior to or subsequent to one or more sintering steps. Co-fire devices will typically be singulated from a larger array multilayer ceramic wafer. A variety of singulation methods are useful to define the geometry of the feedthrough and define the geometric and physical nature of the surfaces of the device. Devices may be singulated in the pre-sintered, or 'green' state, or subsequent to sintering. A variety of methods may be used, including, but not limited to 'green-dicing', laser-cutting, wafer-dicing diamond-saw, wafer-knife, or laser-assisted water-jet. Furthermore, a feedthrough according to the invention can be surrounded or embedded in a suitable potting compound, coated and/or other materials can be applied to one or more surfaces of the structure. This adds to the mechanical and fluidic stability of the device.

The following drawings depict several exemplary embodiments of the invention and are not intended as limiting but rather illustrative of certain aspects of the invention. The drawings are not drawn to scale and common reference numerals are used to denote similar elements of the invention.

## BRIEF DESCRIPTION OF THE DRAWINGS

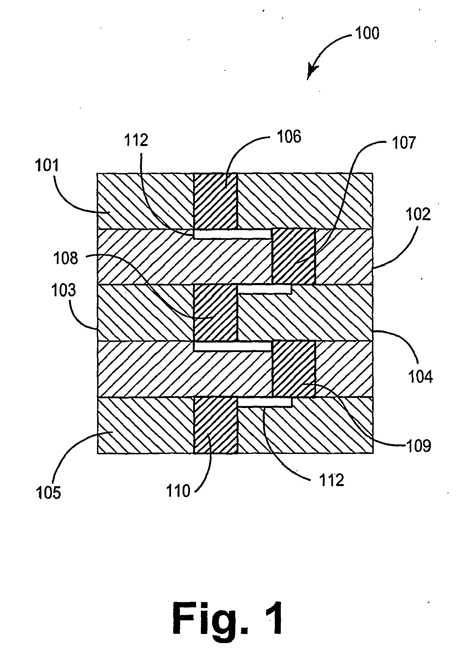

FIG. 1 depicts an elevational side view in cross-section of a hermetic electrical interconnect fabricated using five layers of ceramic green-sheet co-fired to form a monolithic structure with a staggered via structure forming an electrical pathway through said monolithic structure.

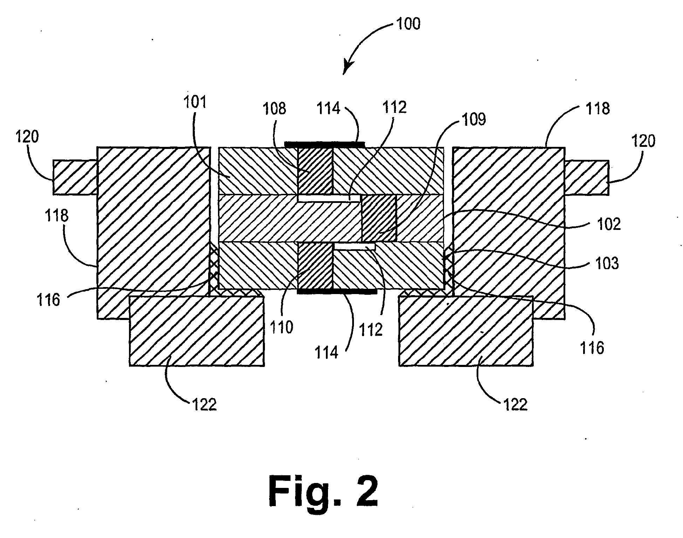

FIG. 2 depicts an elevational side view in cross-section of a co-fired-ceramic hermetic electrical interconnect fabricated using three layers of ceramic green-sheet co-fired to form a monolithic structure with a staggered via structure coupled to a ferrule structure using conventional braze technique. This is shown for background purposes only.

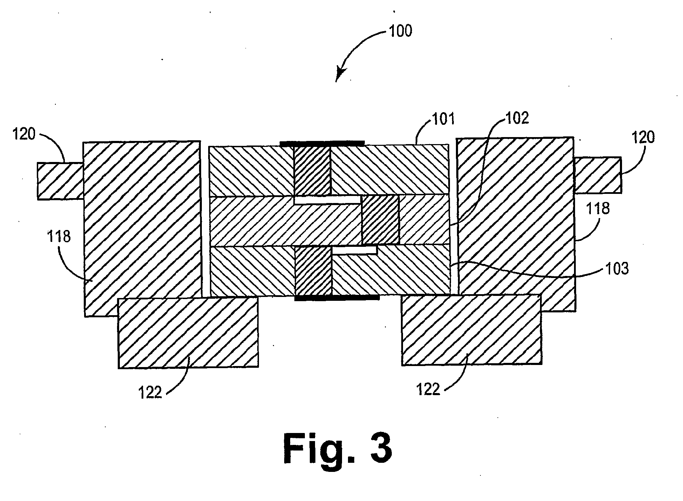

FIG. 3 depicts an elevational side view in cross-section of a co-fire ceramic feedthrough in ferrule, diffusion-bonded with schematic of diffusion-bond interlayer.

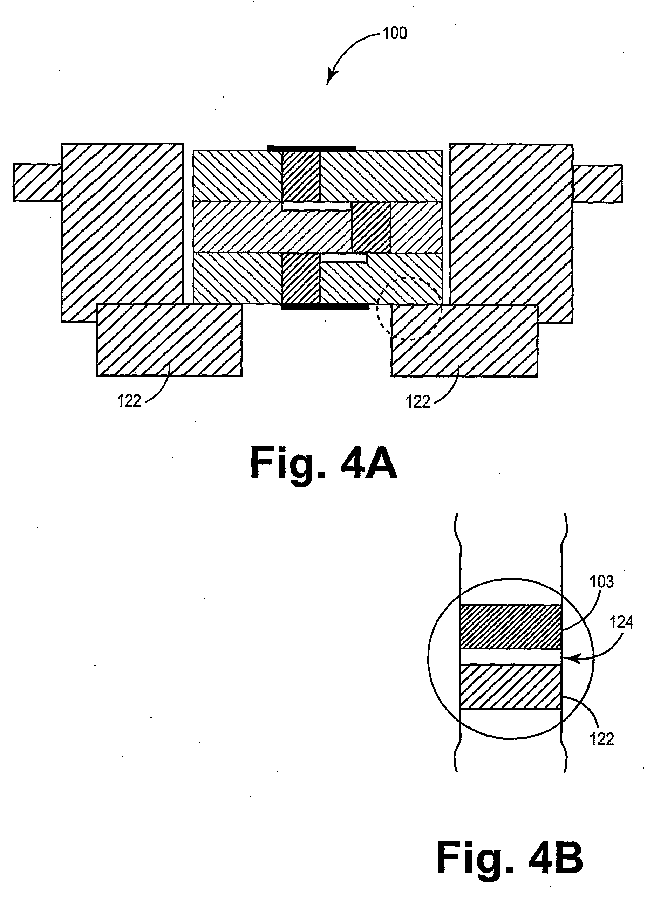

FIG. 4A depicts an elevational side view in cross-section of a co-fired-ceramic hermetic electrical interconnect fabricated using three layers of ceramic green-sheet co-fired to form a monolithic structure with a staggered via structure coupled to a ferrule structure using diffusion-bonding, showing enlarged schematic of diffusion-bond interlayer region.

FIG. 4B is a magnified view of the circular area indicated in FIG. A showing the relative relationship between a monolithic co-fired structure and an underlying support member due to diffusion bonding.

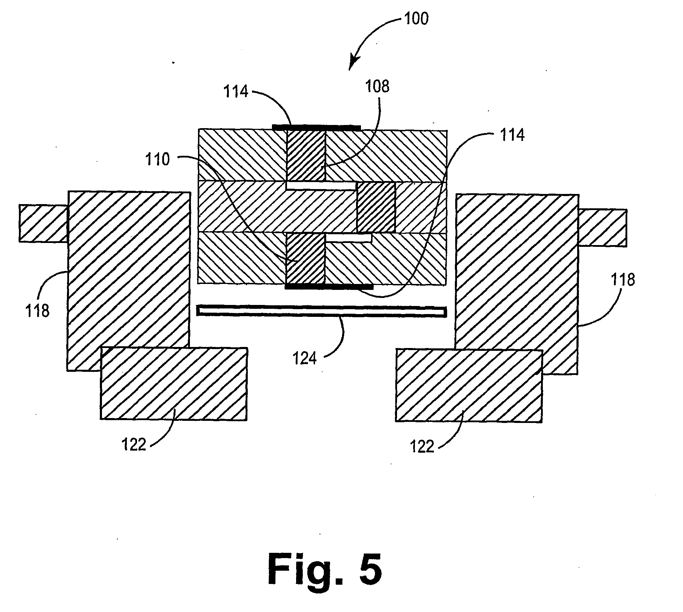

FIG. 5 depicts an elevational side view in cross-section of a co-fired-ceramic hermetic electrical interconnect fabricated using three layers of ceramic green-sheet co-fired to form a monolithic structure with a staggered via structure, with depiction of thin-film reactive interlayer material, and to a ferrule structure prior to stacking, assembly and diffusion-bonding.

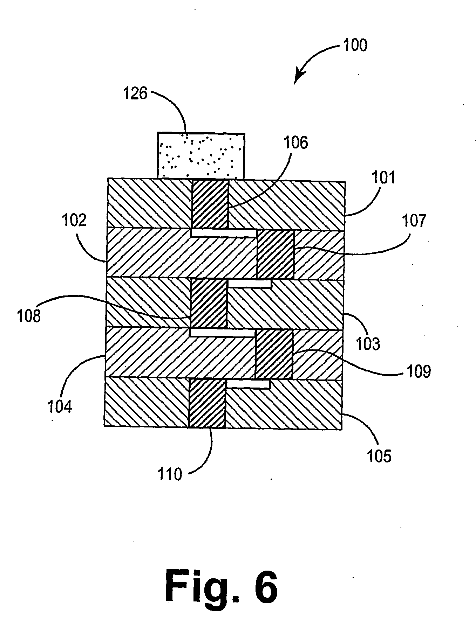

FIG. 6 depicts an elevational side view in cross-section of a hermetic electrical interconnect fabricated using a co-fired voltage-bias resistant, hermetic electrical interconnect fabricated using five layers of ceramic green-sheet co-fired to form a monolithic structure with a staggered via structure forming an electrical pathway from one side of the substrate to the other with diffusion-bonded electrical interconnect structure.

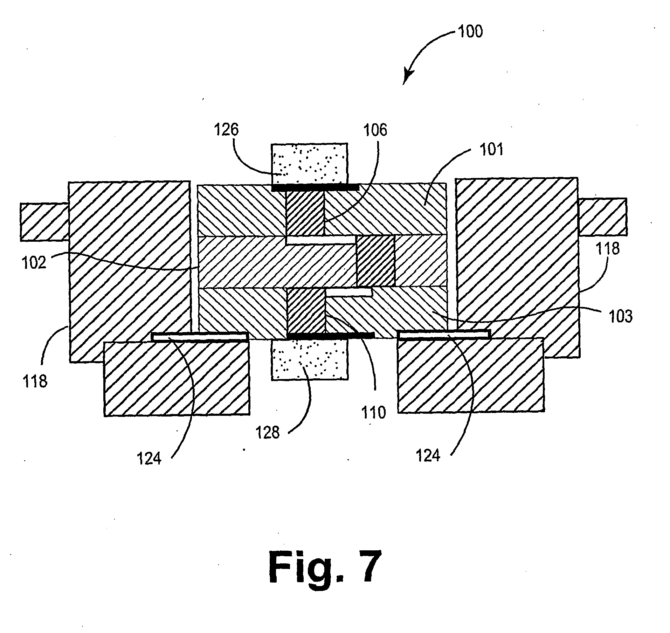

FIG. 7 depicts an elevational side view in cross-section of a hermetic electrical interconnect fabricated a co-fired-ceramic hermetic electrical interconnect fabricated using three layers of ceramic green-sheet co-fired to form a monolithic structure with a staggered via structure coupled to a ferrule structure using diffusion-bonding, with electrical interconnects.

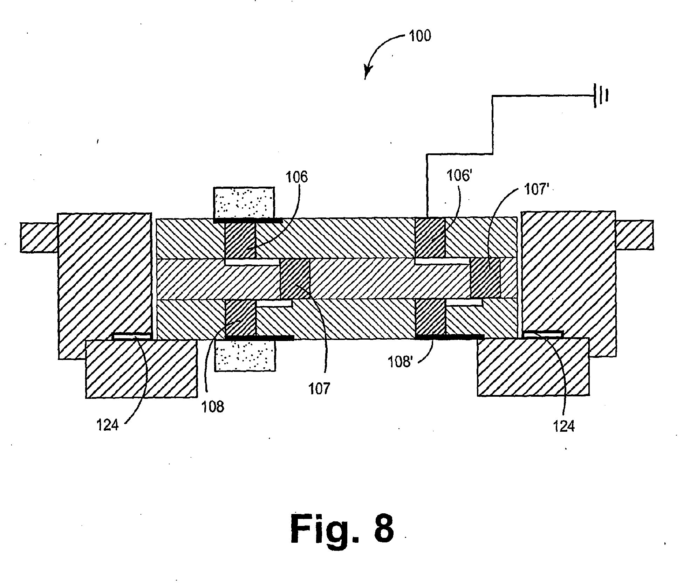

FIG. 8 depicts an elevational side view in cross-section of a hermetic electrical interconnect fabricated with three layers of ceramic green-sheet co-fired to form a monolithic structure with a staggered via structure coupled to a ferrule structure using diffusion-bonding and including a direct ground connection to a conductive ferrule member.

## DETAILED DESCRIPTION OF THE ILLUSTRATED EMBODIMENTS

The following discussion is presented to enable a person skilled in the art to make and use the embodiments of the invention. Various modifications to the illustrated embodiments will be readily apparent to those skilled in the art, and the generic principles herein may be applied to other embodiments and applications without departing from the
scope of the present invention as defined by the appended claims. Thus, the present invention is not intended to be limited to the embodiments shown, but is to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the invention. Skilled artisans will recognize the examples provided herein have many useful alternatives, which fall, within the scope of the invention.

FIG. 1 depicts an elevational side view in cross-section of a hermetic electrical interconnect 100 fabricated using five layers of ceramic green-sheet 101-105 co-fired to form a monolithic structure with a serpentine or staggered, set of via structures 106-110 thus providing a continuous electrical pathway through said five layer monolithic structure 100. The via structures 106-110 are filled with a conductive paste, for example a platinum, platinum-gold, platinum-iridium or other refractory metallic paste, metallic alloy paste, including silver, silver-palladium, gold, gold-palladium or mixtures thereof, tungsten, tungsten-molybdenum, niobium or other refractory metal material to form the a continuous electrical pathway. The structure 100 or a plurality of similar structures can then be sintered or co-fired at elevated temperature to render the structure 100 monolithic and hermetic (as between layer 101 and layer 105). Optionally, one or more conductive interlayers 112 can be disposed in between or adjacent opposing via structures. In the depicted embodiment the interlayers 112 have approximately the same dimension as the corresponding via structure, although different dimensions can be utilized. The interlayer 112 can be formed of the same conductive paste material that is used to fill via structures 106-110 or other conductive material. While the embodiment depicted in FIG. 1 forms a hermetically sealed electrical serpentine pathway through the structure 100, the possibility exists that when subjected to body fluids for an extended period of time the pathway might allow for fluid ingress. By meandering the interconnect using a staggered via geometry the distance that a fluid would have to diffuse into and through the structure 100 is increased thereby increasing its resistance to fluid ingress (due to the length of the conductive pathway), at least relative to a substantially linear geometry. To further enhance the resistance of structure 100 to ingress of body or other fluids, one or more of the interlayer 112 structures can merely abut one or more adjacent vias or optionally fully or partially overlap an end portion of a via. In the embodiment depicted in FIG. 1 a variety of such electrical couplings is depicted. Moreover, an interlayer structure 112 can have a similar or different surface area in contact with a portion of a via depending on whether a particular region of structure 100 needs to increase electrical communication and/or resist fluid intrusion.

Turning now to FIG. 2 which depicts an elevational side view in cross-section is presented for a three-layer hermetic electrical interconnect 100 fabricated using three ceramic green-sheet layers 101-103 co-fired to form a monolithic structure with a staggered via structure 108-110 (and interlayers 112) forming a serpentine electrical pathway through the substrate layers 101,102,103. The depicted embodiment illustrates a variety configuration for interlayer 112 which can include substantially complete coverage for one of the metallized vias (e.g., 108), abutting a side portion of a metallized via (e.g.,

109), and partially covering a metallized via (not depicted). As noted previously, the staggered configuration for the metallized vias 108-110 enhances the hermeticity of the structure 100 as well as increasing the resistance to fluid ingress through the structure 100.

Also depicted in FIG. 2 are a pair of bonding pads 114 providing electrical communication to the vias 108,110 positioned at the exterior of structure 100. In addition to providing a potentially larger bonding surface for connection of remote circuitry the pads 114 increase the resistance of structure 100 to ingress of fluids, such as body fluids. In addition the structure 100 is illustrated inserted into a cavity of a ferrule 118 which in turn is sealingly disposed around the upper periphery of the ferrule 118 within a port of a relatively thin layer of material 120 (e.g., the metallic housing of an IMD). An optionally partially recess lower periphery of the ferrule mechanically couples to form a mechanical stop for the structure 100 when it abuts member 122.

A bonding material 116 is shown disposed intermediate the structure 100 and the ferrule 118 (depicted around the lower periphery of the structure 100). The braze material can comprise a gold (Au) braze or other suitable brazing material.

The ferrule 118 is sized to receive the interconnect 100 and can comprise a metallic member although it can be fabricated of any suitable material including resin and the like. In the event that metal is used to fabricate the ferrule 118 an optional dielectric coating (e.g. oxide or polymer material) can be added to one or more exposed surfaces of the ferrule 118. As depicted and described previously with reference to FIG. 3 the lower support member 122 couples to ferrule 118. Of course, the member 122 can be integrally formed with the ferrule 118 and can be fabricated of a wide variety of materials. Between the ferrule 118, member 122 and the interconnect 100 resides a bonding material 116. In practice the material 116 typically consists of a single material continuously disposed around the periphery of the interconnect 100.

If any open space exists between the material 116, the ferrule 116 and the interconnect 100 then an optional potting compound (not shown) can be applied that will protect the material 116 from direct contact from corrosive body fluid or the like.

Also depicted in FIG. 3 is an edge portion of a sheet of material 120. The material 120 comprises a portion of an enclosure for an IMD, a sensor, an electrochemical cell or other article or component which requires electrical communication. In some forms of the invention the material can comprise titanium, titanium alloys, tantalum, stainless steel, or other metals. Capture pads 114 are coupled to the final metallized vias 108,110 and optional elongated conductors respectively couple to the pads 114.

An embodiment related to the one depicted in FIG. 2 is shown in FIG. 3. Wherein FIG. 3 depicts an elevational side view in cross-section of a three layer hermetic electrical interconnect 100 which is initially fabricated of three layers of ceramic green-sheet 101-103 co-fired to form a monolithic structure with a more complex serpentine via structure forming a continuous electrical pathway through the fired five layer substrate 100. The optional interlayers 112 depicted in FIG. 3 illustrate that they can completely or partially overlap an adjacent metallized via and/or can abut a side portion of a metallized via to establish electrical communication therethrough. One significant difference between the structures depicted involves the use of diffusion bonding in the structure of FIG. 3. The following text provides background and context for the importance of utilizing diffusion bonding techniques in the context of hermetic medical devices.

A diffusion bonding process has a number of advantages over other methods of material-joining, notably: extremely strong/compliant joints. Diffusion bonded joints are particularly pliable yet remain strong and thus are able to endure extremes in temperature. Even where the joined materials have mis-matched thermal expansion coefficients, the joints are totally reliable. Diffusion bonding is therefore particularly suitable for applications threatened by thermal shock at high service temperatures.

Also, diffusion bonded joints are suitable for ultra-high vacuum environments. Solid-phase diffusion bonding utilizes ductile interlayer materials with low outgassing rates to join metallic, ceramic and crystalline materials. The resulting bond is devoid of inclusions and particularly suited to fusion applications.

In addition, in certain situations diffusion bonded joints are reversible. Many of the components produced involve bonding fragile or brittle materials, such as thin optical crystal windows X-ray transparent foils to complex housings and mountings. Should these delicate structures fail or become damaged during use, the bond may in certain instances be reversed so that a replacement window or foil can be fitted to the mounting - a highly satisfactory solution both in terms of economy and the environment.

Thus, diffusion bonding implements a solid-phase process achieved via atomic migration devoid of macro-deformation of the components being joined. Initial surface flatness and cleanliness are essential for a truly hermetic joint to be produced. Surface roughness values of less than about 0.4 microns are generally required and the samples should be cleaned (e.g., in acetone or the like) prior to bonding. Typically the process variables range from several hours at moderate temperatures ($0.6T_m$) to minutes at higher temperatures ($0.8T_m$), with applied pressure. Up until the present time, such diffusion bonding processes are most commonly used for bonding titanium in the aerospace industry. However, ceramics can be diffusion bonded to themselves and/or to other metal materials.

With respect to ceramic bounding, generally a ceramic-ceramic diffusion bond is difficult to achieve unless either diffusion aids or (more commonly) secondary phase materials are present, such as glassy phases at grain boundaries).

Bonding of thin shims to build up complex 3D structures is one of the most up-to-date applications for diffusion bonding technology. This has been achieved for so-called rapid prototyping of alumina components. Some may argue that this is equivalent to sintering, an arguably the two processes are related in the mechanics of diffusion bonding and sintering. Diffusion bonding usually takes place in a uniaxial press (hot isostatic pressing can be used but requires for more complex fixtures) heated using discrete elements or induction units. This also presents a restriction on the size of components that can be processed. However, a more recent innovation uses microwave heating and this has been shown to produce excellent bonds in a matter of minutes, albeit for relatively small components on the order of several inches (e.g., implantable medical devices).

It is also possible to produce ceramic-metal diffusion bonds; and, as for ceramic-ceramic diffusion bonding, a combination of time, temperature and pressure are generally required as the metal deforms at the macro level to the ceramic. Even so, good flat mating surfaces are beneficial. There is oftentimes an added complication when joining dissimilar materials - the differences in coefficient of thermal expansion (CTE). This can cause strains to develop at the interface which can cause premature failure of the bond. This can be overcome by ensuring correct design of the bond line (or surface interface) and/or by using interlayers (as described herein and in the related, co-pending applications), which have CTE's intermediate to those of the materials to be joined and often have a low elastic modulus. Appropriate stacking of interlayers (ranging in thickness from microns to millmeters) can help allow diffusion bonding to successfully occur.

In the electronics industry, alumina and aluminum nitride have been bonded to copper using a thin gold interlayer. Gold is an exceptionally good interlayer and diffusion bonds can be produced at temperatures as low as 300°C although other materials can be utilized.

Diffusion - or arguably more accurately ionic migration - forms the basis of electrostatic bonding. Electrostatic bonding requires that the surfaces being joined must be as flat and clean as possible. The components to be bonded are heated in a vacuum and pressure is applied. Typical conditions would be 3MNm$^{-2}$ and 400°C. When the required temperature has been achieved a DC voltage of about 100V is applied and the metallic component is held to a positive polarity. The nonmetallic component must contain mobile ions such as sodium (Na+). The process has been successfully applied to glass and ceramics such as beta-alumina.

Now returning to the detailed description of the invention and the accompanying drawings, FIG. 3 depicts an elevational side view in cross-section of a co-fire ceramic feedthrough 100 disposed within a surrounding ferrule 118. The ferrule 118 is mechanically sized to fit tightly within an aperture formed in the substrate 120 (e.g., a portion of an enclosure of an IMD). As depicted in FIG. 2, a lower member 122 abuts ferrule 118 and provide mechanical support and structure overlapping both the ferrule 118 and the lower portion of feedthrough 100.

The structure depicted in FIG. 4A and FIG. 4B illustrates the location of a diffusion-bonded region between the ferrule 118 and the member 122 (encircled and enlarged in FIG. 4B) as a schematic of a diffusion-bond interlayer 124. As depicted in FIG. 2 (but not in FIG. 4A or 4B), the space or location above ferrule 118 and the feedthrough 100 can optionally include a high temperature brazed seal, as previously described.

FIG. 5 depicts an elevational side view in cross-section of a co-fired-ceramic. hermetic electrical interconnect fabricated using three layers of ceramic green-sheet co-fired to form a monolithic structure with a staggered via structure, with depiction of thin-film reactive material forming interlayer 124 and to a ferrule structure prior to stacking the feedthrough 100, structure 122 and ferrule 118. And, also prior to assembly of the components and diffusion-bonding of the structure 122 and the feedthrough 100. The interlayer 124 can be formed with an aperture or apertures (not shown) that correspond to one or more capture pads 114 or surface portions of one or more via structures 108,110 disposed on an exterior portion of the feedthrough 100.

FIG. 6 depicts an elevational side view in cross-section of a hermetic electrical interconnect 100 using five layers of ceramic green-sheet material 101-105 co-fired to form a monolithic structure with a staggered via structure 106-110 forming a continuous electrical pathway from one side of the substrate to the other with a diffusion-bonded electrical interconnect structure 126 disposed on a upper surface of the upper layer 101. As depicted, the interconnect structure 126 overlays the upper otherwise exposed surface of via 106 thereby providing additional fluid impedance or resistance to ingress or flow of fluid into the feedthrough 100.

FIG. 7 depicts an elevational side view in cross-section of a hermetic electrical interconnect 100 fabricated using three layers of ceramic green-sheet 101-103 co-fired to form a monolithic structure with a staggered via structure coupled to a ferrule structure 118 using diffusion-bonding techniques. The interconnect 100 includes electrical interconnect structures 126,128 coupled to via structures 106,110 disposed at opposing sides of the interconnect 100. Among other advantages, the electrical interconnect structures 126,128 enhance surface area and mechanical integrity for bonding of conductive elements thereto and can serve as fiducial alignment posts to aid automated fabrication and/or electrical couplings to said feedthrough 100.

FIG. 8 depicts an elevational side view in cross-section of a hermetic electrical interconnect 100 fabricated a co-fired-ceramic hermetic electrical interconnect fabricated using three layers of ceramic green-sheet 101-103 co-fired to form a monolithic structure with a pair of staggered via structures 106-108 and 106'-108' coupled to a ferrule structure 118 using diffusion-bonding, with electrical interconnecting structures 126,128 and including a direct-to-ground connection to the ferrule structure 118.

Thus, embodiments of the IMPLANTABLE CO-FIRED ELECTRICAL FEEDTHROUGHS are disclosed. One skilled in the art will appreciate that the invention can be practiced with embodiments other than those disclosed. The disclosed embodiments are presented for purposes of illustration and not limitation, and the invention is limited only by the claims that follow.

**Claims**

1. A miniaturized hermetic electrical interconnect for an implantable medical device (IMD), comprising:

   a monolithic structure derived from at least three discrete ceramic green-state sheet layers (101-105) with each said at least three ceramic green-state sheet layers having at least one via-receiving aperture (106-110) coupling major planar sides thereof;
   a conductive metallic material (112) disposed within and at least partially filling each of the via-receiving apertures;
   a ferrule structure (118) surrounding the monolithic structure, said ferrule structure having an upper surface and a lower surface; and
   a structural support member (122) coupled to at least a portion of the lower surface and a lower portion of said monolithic structure,
   wherein said monolithic structure and said conductive metallic material are hermetically fused together at elevated temperature,
   wherein the coupling between said structural support member and said monolithic structure comprises a diffusion bond.

2. An interconnect according to claim 1, wherein said monolithic structure comprises an insulating dielectric; said insulating dielectric selected from a group consisting of: a Al2O3 material, a Al2O3-ZrO2 material, ZrO2, a glass material.

3. An interconnect according to claim 1, wherein the elevated temperature comprises a temperature of between about 650 degrees C. and 1300 degrees C.

4. An interconnect according to claim 1, wherein the conductive metallic material comprises at least one of: a platinum material, a platinum-gold material, a platinum-iridium material, a platinum alloy material, a tungsten material, a tungsten-molybdenum material, a niobium material, a silver material, a gold material, a silver-palladium material, a gold-palladium material.

5. An interconnect according to claim 1, further comprising a braze-metal bond intermediate the ferrule structure and the monolithic structure

6. An interconnect according to claim 1, wherein the braze-metal bond comprises a gold material.

7. An interconnect according to claim 1, further comprising at least one capture pad coupled to the conductive metallic material disposed within one the via-receiving apertures.

8. An interconnect according to claim 1, wherein the via-receiving apertures are disposed in substantially axial alignment.

9. An interconnect according to claim 1, wherein the via-receiving apertures of adjacent green-state sheet layers are offset laterally and further comprising a conductive material coupling said offset via-receiving apertures.

10. An interconnect according to claim 1, wherein said capture pad electrically couples to an electrical circuit producing at least temporarily an electrical voltage-bias signal.

11. An interconnect according to claim 1, wherein said capture pad comprises at least one of: a platinum material, a platinum-gold material, a platinum-iridium material, a platinum alloy material, a tungsten material, a tungsten-molybdenum material, a niobium material, a silver material, a gold material, a silver-palladium material, a gold-palladium material.

12. An interconnect according to claim 1, wherein said capture pad comprises a thin-film structure.

13. An interconnect according to claim 1, wherein said capture pad comprises at least one of: a niobium material, a tantalum material, a titanium material, a platinum material, a platinum alloy material.

14. An interconnect according to claim 1, wherein said capture pad comprises a thick-film metallization ink applied subsequent to the co-firing of the metallic paste and the green-state sheet layers

15. An interconnect according to claim 1, wherein at least one layer of the at least three discrete ceramic green-sheet layers comprises a low temperature co-fire ceramic (LTCC) material.

16. An interconnect according to claim 15, wherein the LTCC material has a melting point between about 850 degrees Celsius and 1150 degrees Celsius.

**17.** An interconnect according to claim 1, wherein at least one layer of the at least three discrete ceramic green-sheet layers comprises a high temperature co-fire ceramic (HTCC) material.

**18.** An interconnect according to claim 17, wherein the HTCC material comprises a refractory metal material.

**19.** An interconnect according to claim 18, wherein the HTCC material has a melting point between about 1100 degrees Celsius and 1700 degrees Celsius

**20.** An interconnect according to claim 1, wherein said ferrule structure comprises at least one of: a titanium material, a titanium alloy material, a ceramic oxide material, a polymer material,

**21.** An interconnect according to claim 20, wherein the polymer material comprises a polyetheretherketone material.

**22.** An interconnect according to claim 1, further comprising an aperture formed through a portion of an enclosure, said aperture configured to sealingly receive peripheral edges of the ferrule structure;

**23.** An interconnect according to claim 1, wherein said enclosure contains an electrochemical cell

**24.** An interconnect according to claim 23, wherein said electrochemical cell comprises one of: a primary battery, a secondary batter, a capacitor.

**25.** An interconnect according to claim 1, wherein said enclosure contains at least a part of an implantable medical device (IMD).

**26.** An interconnect according to claim 25, wherein said IMD comprises one of: a pacemaker, a neurological stimulator, a drug pump, a cardioverter-defibrillator, a deep brain stimulator, a medical electrical lead, a physiologic sensor.

**Patentansprüche**

**1.** Miniaturisierte, hermetische elektrische Zwischenverbindung für eine implantierbare medizinische Vorrichtung (IMD), mit:

einer monolithischen Struktur, die aus wenigstens drei diskreten Rohzustand-Keramiklagenschichten (101-105) abgeleitet ist, wobei jede der wenigstens drei Rohzustand-Keramiklagenschichten wenigstens eine Durchgangskopplungs-Aufnahmeöffnung (106-110) besitzt, die ebene Hauptseiten hiervon koppelt;

einem leitenden Metallmaterial (112), das in jeder der Durchgangskopplungs-Aufnahmeöffnungen angeordnet ist und diese wenigstens teilweise füllt;

einer Hülsen- bzw. Ringbeschlagstruktur (118), die die monolithische Struktur umgibt, wobei die Ringbeschlagstruktur eine obere Oberfläche und eine untere Oberfläche besitzt; und

einem strukturellen Tragelement (122), das wenigstens mit einem Abschnitt der unteren Oberfläche und mit einem unteren Abschnitt der monolithischen Struktur gekoppelt ist,

wobei die monolithische Struktur und das leitende Metallmaterial bei erhöhter Temperatur hermetisch verschmolzen sind,

wobei die Kopplung zwischen dem strukturellen Tragelement und der monolithischen Struktur eine Diffusionshaftung aufweist.

**2.** zwischenverbindung nach Anspruch 1, wobei die monolithische Struktur ein isolierendes Dielektrikum enthält; wobei das isolierende Dielektrikum aus einer Gruppe gewählt ist, die besteht aus: einem $Al_2O_3$-Material, einem $Al_2O_3$-$ZrO_2$-Material, aus $ZrO_2$, aus einem Glasmaterial.

**3.** Zwischenverbindung nach Anspruch 1, wobei die erhöhte Temperatur eine Temperatur im Bereich von etwa 650 Grad C bis 1300 Grad C enthält.

**4.** Zwischenverbindung nach Anspruch 1, wobei das leitende Metallmaterial wenigstens eines der Folgenden enthält: ein Platin-Material, ein Platin-Gold-Material, ein Platin-Iridium-Material, ein Platinlegierungs-Material, ein Wolfram-Material, ein Wolfram-Molybdän-Material, ein Niob-Material, ein Silber-Material, ein Gold-Material, ein Silber-Palladium-Material, ein Gold-Palladium-Material.

**5.** Zwischenverbindung nach Anspruch 1, die ferner zwischen der Ringbeschlagstruktur und der monolithischen Struktur eine Hartlöt-Metallverbindung aufweist.

**6.** Zwischenverbindung nach Anspruch 1, wobei die Hartlöt-Metallverbindung ein Gold-Material enthält.

**7.** Zwischenverbindung nach Anspruch 1, die ferner wenigstens eine Aufnahmeanschlussfläche aufweist, die mit dem leitenden Metallmaterial gekoppelt ist und in einer der Durchgangskopplungs-Aufnahmeöffnungen angeordnet ist.

**8.** Zwischenverbindung nach Anspruch 1, wobei die Durchgangskopplungs-Aufnahmeöffnungen im Wesentlichen axial ausgerichtet angeordnet sind.

9. Zwischenverbindung nach Anspruch 1, wobei die Durchgangskopplungs-Aufnahmeöffnungen benachbarter Rohzustand-Lagenschichten seitlich versetzt sind und ferner ein leitendes Material aufweisen, das die versetzten Durchgangskopplungs-Aufnahmeöffnungen koppelt.

10. Zwischenverbindung nach Anspruch 1, wobei die Aufnahmeanschlussfläche mit einer elektrischen Schaltung, die wenigstens zeitweilig ein elektrisches Vorspannungssignal erzeugt, elektrisch gekoppelt ist.

11. Zwischenverbindung nach Anspruch 1, wobei die Aufnahmeanschlussfläche wenigstens eines der folgenden Materialien enthält: ein Platin-Material, ein Platin-Gold-Material, ein Platin-Iridium-Material, ein Platinlegierungs-Material, ein Wolfram-Material, ein Wolfram-Molybdän-Material, ein Niob-Material, ein Silber-Material, ein Gold-Material, ein Silber-Palladium-Material, ein Gold-Palladium-Material.

12. Zwischenverbindung nach Anspruch 1, wobei die Aufnahmeanschlussfläche eine Dünnschichtstruktur aufweist.

13. Zwischenverbindung nach Anspruch 1, wobei die Aufnahmeanschlussfläche wenigstens eines der folgenden Materialien enthält: ein Niob-Material, ein Tantal-Material, ein Titan-Material, ein Platin-Material, ein Platinlegierungs-Material.

14. Zwischenverbindung nach Anspruch 1, wobei die Aufnahmeanschlussfläche eine Dickschicht-Metallisierungstinte enthält, die nach dem gemeinsamen Erhitzen der Metallpaste und der Rohzustand-Lagenschichten aufgebracht wird.

15. Zwischenverbindung nach Anspruch 1, wobei wenigstens eine Schicht der wenigstens drei diskreten Rohzustand-Keramikschichten ein Niedrigtemperatur-Gemeinsamerhitzungs-Keramikmaterial (LTCC-Material) enthält.

16. Zwischenverbindung nach Anspruch 15, wobei das LTCC-Material einen Schmelzpunkt im Bereich von etwa 850 Grad Celsius bis 1150 Grad Celsius besitzt.

17. Zwischenverbindung nach Anspruch 1, wobei wenigstens eine Schicht der wenigstens drei diskreten Rohzustand-Keramiklagenschichten ein Hochtemperatur- Gemeinsamerhitzungs- Keramikmaterial (HTTC-Material) enthält.

18. Zwischenverbindung nach Anspruch 17, wobei das HTTC-Material ein hitzebeständiges Metallmaterial enthält.

19. Zwischenverbindung nach Anspruch 18, wobei das HTTC-Material einen Schmelzpunkt im Bereich von etwa 1100 Grad Celsius bis 1700 Grad Celsius besitzt.

20. Zwischenverbindung nach Anspruch 1, wobei die Ringbeschlagstruktur wenigstens eines der folgenden Materialien enthält: ein Titan-Material, ein Titanlegierungs-Material, ein Keramikoxid-Material, ein Polymer-Material.

21. Zwischenverbindung nach Anspruch 20, wobei das Polymer-Material ein Polyetheretherketon-Material enthält.

22. Zwischenverbindung nach Anspruch 1, die ferner eine Öffnung aufweist, die durch einen Abschnitt eines Gehäuses ausgebildet ist, wobei die Öffnung konfiguriert ist, um Umfangskanten der Ringbeschlagstruktur abdichtend aufzunehmen.

23. Zwischenverbindung nach Anspruch 1, wobei das Gehäuse eine elektrochemische Zelle enthält.

24. Zwischenverbindung nach Anspruch 23, wobei die elektrochemische Zelle eines der Folgenden enthält: eine Primärbatterie, eine Sekundärbatterie, einen Kondensator.

25. Zwischenverbindung nach Anspruch 1, wobei das Gehäuse wenigstens einen Teil einer implantierbaren medizinischen Vorrichtung (IMD) enthält.

26. Zwischenverbindung nach Anspruch 25, wobei die IMD eines der Folgenden enthält: einen Herzschrittmacher, einen neurologischen Stimulator, eine Arzneimittelpumpe, einen Kardioverter-Defibrillator, einen Tiefengehirnstimulator, eine medizinische elektrische Leitung, einen physiologischen Sensor.

**Revendications**

1. Interconnexion électrique hermétique miniaturisée pour un dispositif médical implantable (IMD), comportant :

   une structure monolithique dérivée d'au moins trois couches de feuille d'état vert en céramique discrètes (101 - 105), chacune desdites au moins trois couches de feuille d'état vert en céramique ayant au moins une ouverture de réception de trou d'interconnexion (106 - 110) couplant des côtés planaires principaux correspondants ;
   un matériau métallique conducteur (112) disposé dans chacune des ouvertures de réception de trou d'interconnexion et remplissant au moins partiellement chacune d'entre elles;

une structure de type férule (118) entourant la structure monolithique, ladite structure de type férule ayant une surface supérieure et une surface inférieure ; et

un élément de support structurel (122) couplé à au moins une partie de la surface inférieure et à une partie inférieure de ladite structure monolithique,

dans laquelle ladite structure monolithique et ledit matériau métallique conducteur sont hermétiquement fondus ensemble à une température élevée,

le couplage entre ledit élément de support structurel et ladite structure monolithique comportant un soudage par diffusion.

2. Interconnexion selon la revendication 1, dans laquelle ladite structure monolithique comporte un diélectrique isolant ; ledit diélectrique isolant étant sélectionné à partir d'un groupe constitué : d'un matériau A1203, d'un matériau A1203-ZrO2, Zr02, d'un matériau en verre.

3. Interconnexion selon la revendication 1, dans laquelle la température élevée comprend une température comprise entre 650°C et 1300 °C.

4. Interconnexion selon la revendication 1, dans laquelle le matériau métallique conducteur comporte au moins un matériau parmi : un matériau en platine, un matériau en platine - or, un matériau en platine - iridium, un matériau en alliage de platine, un matériau en tungstène, un matériau en tungstène - molybdène, un matériau en niobium, un matériau en argent, un matériau en or, un matériau en argent - palladium, un matériau en or - palladium.

5. Interconnexion selon la revendication 1, comportant en outre une liaison par métal de brasage intermédiaire entre la structure de type férule et la structure monolithique.

6. Interconnexion selon la revendication 1, dans laquelle la liaison par métal de brasage comporte un matériau en or.

7. Interconnexion selon la revendication 1, comportant en outre au moins une pastille de capture couplée au matériau métallique conducteur disposé dans l'une des ouvertures de réception de trou d'interconnexion.

8. Interconnexion selon la revendication 1, dans laquelle les ouvertures de réception de trou d'interconnexion sont disposées selon un alignement sensiblement axial.

9. Interconnexion selon la revendication 1, dans laquelle les ouvertures de réception de trou d'interconnexion de couches de feuille d'état vert adjacentes sont décalées latéralement et comportant en outre un matériau conducteur couplant lesdites ouvertures de réception de trou d'interconnexion décalées.

10. Interconnexion selon la revendication 1, dans laquelle ladite pastille de capture est électriquement couplée à un circuit électrique produisant au moins temporairement un signal de tension de polarisation électrique.

11. Interconnexion selon la revendication 1, dans laquelle ladite pastille de capture comporte au moins un matériau parmi : un matériau en platine, un matériau en platine - or, un matériau en platine - iridium, un matériau en alliage de platine, un matériau en tungstène, un matériau en tungstène - molybdène, un matériau en niobium, un matériau en argent, un matériau en or, un matériau en argent - palladium, un matériau en or - palladium.

12. Interconnexion selon la revendication 1, dans laquelle ladite pastille de capture comporte une structure en film mince.

13. Interconnexion selon la revendication 1, dans laquelle ladite pastille de capture comporte au moins un matériau parmi : un matériau en niobium, un matériau en tantale, un matériau en titane, un matériau en platine, un matériau en alliage de platine.

14. Interconnexion selon la revendication 1, dans laquelle ladite pastille de capture comporte une encre de métallisation de film épais appliquée à la suite de la cocuisson de la pâte métallique et des couches de feuille d'état vert.

15. Interconnexion selon la revendication 1, dans laquelle au moins une couche parmi les au moins trois couches de feuille d'état vert en céramique discrètes comporte un matériau en céramique cocuit à basse température (LTCC).

16. Interconnexion selon la revendication 15, dans laquelle le matériau LTCC a un point de fusion compris entre environ 850°C et 1150 °C.

17. Interconnexion selon la revendication 1, dans laquelle au moins une couche parmi les au moins trois couches de feuille d'état vert en céramique discrètes comporte un matériau en céramique cocuit à température élevée (HTCC).

18. Interconnexion selon la revendication 17, dans laquelle le matériau HTCC comporte un matériau en métal réfractaire.

**19.** Interconnexion selon la revendication 18, dans laquelle le matériau HTTC a un point de fusion compris entre environ 1100 °C et 1700 °C.

**20.** Interconnexion selon la revendication 1, dans laquelle ladite structure de type férule comporte au moins un matériau parmi : un matériau en titane, un matériau en alliage de titane, un matériau en oxyde de céramique, un matériau polymère.

**21.** Interconnexion selon la revendication 20, dans laquelle le matériau polymère comporte un matériau en polyétheréthercétone.

**22.** Interconnexion selon la revendication 1, comportant en outre une ouverture formée à travers une partie d'une enceinte, ladite ouverture étant configurée pour recevoir hermétiquement des bords périphériques de la structure de type férule.

**23.** Interconnexion selon la revendication 1, dans laquelle ladite enceinte contient une cellule électrochimique.

**24.** Interconnexion selon la revendication 23, dans laquelle ladite cellule électrochimique comporte l'une parmi : une batterie principale, une batterie secondaire, un condensateur.

**25.** Interconnexion selon la revendication 1, dans laquelle ladite enceinte contient au moins une partie d'un dispositif médical implantable (IMD).

**26.** Interconnexion selon la revendication 25, dans laquelle ledit dispositif médical implantable comporte au moins l'un parmi : un stimulateur cardiaque, un stimulateur neurologique, une pompe à médicaments, un cardioverteur - défibrillateur, un stimulateur cérébral profond, une dérivation électrique médicale, un capteur physiologique.

**Fig. 1**

# Fig. 2

**Fig. 3**

100

122

122

# Fig. 4A

103

124

122

# Fig. 4B

**Fig. 5**

**Fig. 6**

**Fig. 7**

Fig. 8

**EP 1 934 994 B1**

## REFERENCES CITED IN THE DESCRIPTION

### Patent documents cited in the description

- US 6743534 B, Lautzenhiser
- US 4654095 A, Steinberg
- US 4641425 A, Dubuisson
- US 4910643 A, Williams
- US 4464420 A, Taguchi
- US 5356841 A, Mitzutani
- US 5831810 A, Bird
- US 5855995 A
- US 5821181 A, Ursula
- US 5102720 A
- US 4340436 A
- EP 0243858 A
- US 6139666 A
- US 6205032 B
- US 6560860 B
- US 5085720 A
- US 5254191 A
- US 5383474 A
- US 5474741 A, Mikeska